**Europäisches Patentamt**

**European Patent Office**

**Office européen des brevets**

(19)

(11) Veröffentlichungsnummer: **0 542 106 B1**

(12) ## EUROPÄISCHE PATENTSCHRIFT

(45) Veröffentlichungstag der Patentschrift: **27.12.95**

(51) Int. Cl.⁶: **C07C 263/04**

(21) Anmeldenummer: **92118832.2**

(22) Anmeldetag: **03.11.92**

(54) **Verfahren zur Herstellung von Polyisocyanaten**

(30) Priorität: **14.11.91 DE 4137428**

(43) Veröffentlichungstag der Anmeldung:
**19.05.93 Patentblatt 93/20**

(45) Bekanntmachung des Hinweises auf die
Patenterteilung:
**27.12.95 Patentblatt 95/52**

(84) Benannte Vertragsstaaten:
**BE DE ES FR GB IT NL**

(56) Entgegenhaltungen:
**EP-A- 0 003 570**
**EP-A- 0 143 320**

(73) Patentinhaber: **BAYER AG**

**D-51368 Leverkusen (DE)**

(72) Erfinder: **Hauner, Andreas, Dr.**
**Ehreshover Strasse 14**
**W-5000 Köln 60 (DE)**
Erfinder: **Hennig, Hans-Joachim, Dr.**
**Saarbrücker Strasse 42**
**W-5090 Leverkusen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 542 106 B1

**Beschreibung**

Die Erfindung betrifft ein neues Verfahren zur kontinuierlichen Herstellung von organischen Polyisocyanaten, insbesondere Diisocyanaten durch thermische Spaltung der ihnen zugrundeliegenden Carbamidsäureester.

Es ist seit langen Jahren bekannt, daß N-substituierte Urethane in Isocyanate und Alkohol spaltbar sind [H. Schiff, Ber. Dtsch. Chem. Ges. 3, 649 (1870); A.W. Hoffmann, Ber. Dtsch. Chem. Ges. 3, 653 (1870)].

Die EP-A-0 143 320 beschreibt beispielsweise ein derartiges Verfahren bei vorzugsweise hohen Temperaturen von 360 - 440° C, wobei gegebenenfalls vergleichsweise niedrigsiedende Lösungsmittel mitverwendet werden können, deren Siedepunkt zwischen denen der entsprechenden Isocyanate und Alkohole liegt. Sehr wahrscheinlich wegen der hohen Temperaturbelastung liegen die konkret in den Beispielen mitgeteilten Ausbeuten, bezogen auf eingesetztes Urethan, deutlich unter 90 % der Theorie.

Bereits vor geraumer Zeit wurde jedoch gefunden, daß die Spaltung vorzugsweise in Gegenwart von hochsiedenden Lösungsmitteln für die Carbamidsäureester erfolgt. So beschreibt beispielsweise die US-PS 3 919 278 ein Verfahren zur Herstellung von aromatischen Polyisocyanaten in einem Spaltreaktor mit aufgesetzter Destillationskolonne unter Mitverwendung derartiger Hilfslösungsmittel und gleichzeitiger Mitverwendung eines Trägergases zwecks besserer Ausschleusung der bei der Spaltung entstehenden Spaltprodukte aus dem Reaktionsgemisch.

Die US-PS 3 962 302 beschreibt ein ähnliches Verfahren. Beiden Verfahren gemeinsam sind verhältnismäßig schlechte Ausbeuten, was vermutlich darauf zurückzuführen ist, daß sich die als Spaltprodukte anfallenden Polyisocyanate nur schwer aus dem Spaltmedium abtrennen lassen. Vermutlich wird aus diesem Grund gemäß DE-OS 2 530 001 des gleichen Anmelders das Spaltmedium einer separaten Aufarbeitung unterzogen, um das darin enthaltende Diisocyanat zu gewinnen.

Außer diesen in Abwesenheit von Katalysatoren durchzuführenden Verfahren wurden auch Verfahren vorgeschlagen, bei denen die Ausbeute durch Mitverwendung der unterschiedlichsten Katalysatoren erhöht werden soll. Einschlägige Vorveröffentlichungen sind beispielsweise US-PS 3 919 279, DE-OS 2 635 490, DE-OS 2 942 543 oder EP-A-0 323 514. Es versteht sich, daß die Mitverwendung von Katalysatoren im Vergleich zu einem katalysatorfreien Verfahren mit bestimmten Nachteilen behaftet ist. So kann beispielsweise nie völlig ausgeschlossen werden, daß Katalysator-Spuren in die Verfahrensprodukte gelangen und deren Eigenschaften in unerwünschter Weise verändern, oder daß die Aufarbeitung des Hilfslösungsmittels durch die Anwesenheit der Katalysatoren erschwert wird.

Es war daher die der Erfindung zugrundeliegende Aufgabe, ein neues Verfahren zur Herstellung von organischen Polyisocyanaten, insbesondere Diisocyanaten durch Spaltung der entsprechenden Carbamidsäureester zur Verfügung zu stellen, welches auf einfache Weise die kontinuierliche, katalysatorfreie Herstellung der Diisocyanate in hohen Ausbeuten gestattet.

Diese Aufgabe konnte mit der Bereitstellung des nachstehend näher beschriebenen erfindungsgemäßen Verfahrens gelöst werden.

Gegenstand der Erfindung ist ein Verfahren zur kontinuierlichen, katalysatorfreien Herstellung von Polyisocyanaten durch thermische Spaltung der den Polyisocyanaten entsprechenden N-substituierten Carbamidsäureester bei 200° C bis 300° C in einer Sumpf-beheizten, als Spaltreaktor dienenden Destillationskolonne unter anschließender Auftrennung der Spaltprodukte in eine, vorwiegend aus der Isocyanatkomponente bestehende Fraktion I und eine vorwiegend aus der Alkoholkomponente der Carbamidsäureester bestehende Fraktion II, wobei die Spaltung in Gegenwart eines Hochsieders durchgeführt wird, der für die Carbamidsäureester ein Lösungsmittel darstellt, gegenüber den Carbamidsäureestern und den Spaltprodukten inert ist, unter den Druck- und Temperaturbedingungen im Sumpf der Destillationskolonne siedet und unter diesen Druckbedingungen einen Siedepunkt aufweist, der mindestens 10° C über dem Siedepunkt beider Spaltprodukte liegt, dadurch gekennzeichnet, daß die zu spaltenden Carbamidsäureester in Form einer 5 bis 90-gew.-%igen Lösung oberhalb des Spaltdrucks auf eine Temperatur innerhalb des Bereichs von 100°C bis 300° C, jedoch eine Temperatur, die oberhalb des Schmelzpunktes des Polyurethans liegt, erhitzt werden und anschließend unter Entspannung als Seitenstrom in die Destillationskolonne (4) geleitet werden, in deren Sumpf ein Druck (Spaltdruck) innerhalb des Bereichs von 0,001 bis 5 bar und eine Temperatur innerhalb des Bereichs von 200° C bis 300° C so aufrecht erhalten werden, daß der Hochsieder im Sumpf am Sieden gehalten wird, wobei gleichzeitig die Spaltprodukte am Kopf der Destillationskolonne in Form der Fraktionen I und II kontinuierlich selektiv kondensiert und über den Sumpfablauf kontinuierlich eine solche Menge des gegebenenfalls Verunreinigungen enthaltenden Hochsieders ausgeschleust werden, die in erster Näherung der Menge des Hochsieders entspricht, der als Lösungsmittel für den Carbamidsäureester in die Kolonne eingespeist wird.

2

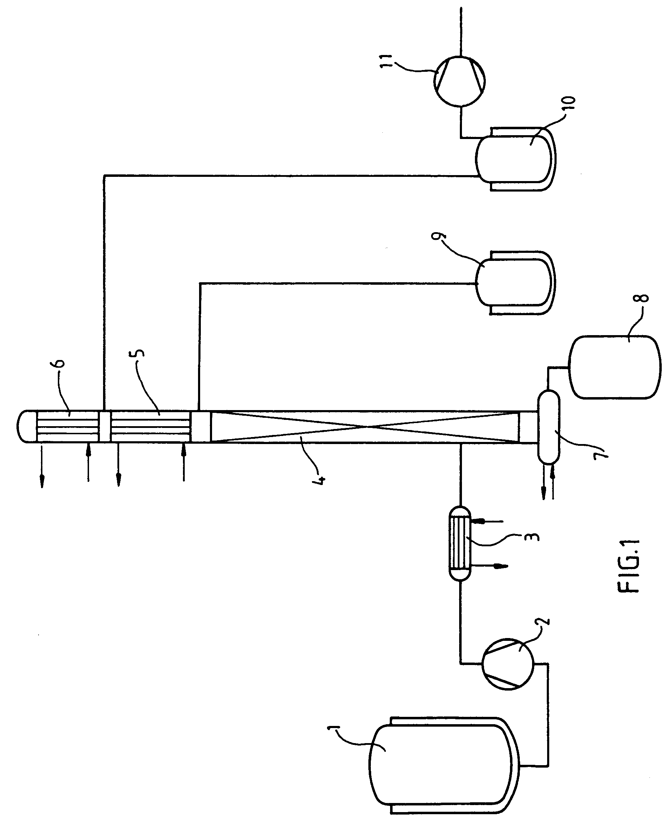

Das Verfahren kann beispielsweise in der aus der Zeichnung ersichtlichen Apparatur durchgeführt werden. In dieser Zeichnung bedeuten im einzelnen

(1) einen beheizbaren Vorratsbehälter für die zu spaltende Carbamidsäureester-Lösung;

(2) eine beheizbare Dosierpumpe für diese Lösung;

(3) einen Wärmeaustauscher zur Vorerhitzung der Lösung der zu spaltenden Carbamidsäureester;

(4) die als Spaltreaktor dienende Destillationskolonne;

(5) einen Dephlegmator;

(6) einen Kondensator;

(7) die Sumpfbeheizung, beispielsweise in Form eines Umlauferhitzers;

(8) den Sumpf-Ablaufbehälter;

(9) den Vorratsbehälter für die Polyisocyanatfraktion I;

(10) den mit einer Kühlvorrichtung versehenen Vorratsbehälter für die Alkoholfraktion II und

(11) eine Vakuumpumpe.

Bei den beim erfindungsgemäßen Verfahren einzusetzenden Carbamidsäureestern handelt es sich um Verbindungen der allgemeinen Formel $R^1(NHCOOR^2)_n$.

In dieser Formel stehen

$R^1$ für einen gegebenenfalls inerte Substituenten aufweisenden aliphatischen Kohlenwasserstoffrest mit insgesamt 4 bis 12 Kohlenstoffatomen, einen gegebenenfalls inerte Substituenten aufweisenden cycloaliphatischen Kohlenwasserstoffrest mit insgesamt 6 bis 15 Kohlenstoffatomen, einen gegebenenfalls inerte Substituenten aufweisenden araliphatischen Kohlenwasserstoffrest mit insgesamt 7 bis 10 Kohlenstoffatomen oder einen gegebenenfalls inerte Substituenten aufweisenden aromatischen Kohlenwasserstoffrest mit insgesamt 6 bis 15 Kohlenstoffatomen,

$R^2$ für einen aliphatischen Kohlenwasserstoffrest mit 1 bis 20 Kohlenstoffatomen, einen cycloaliphatischen Kohlenwasserstoffrest mit 5 bis 15 Kohlenstoffatomen oder einen aromatischen Kohlenwasserstoffrest mit 6 bis 15 Kohlenstoffatomen und

n für eine ganze Zahl von 2 bis 5.

Vorzugsweise werden beim erfindungsgemäßen Verfahren solche Carbamidsäureester der genannten Formel eingesetzt, für welche

$R^1$ für einen aliphatischen Kohlenwasserstoffrest mit insgesamt 4 bis 12, insbesondere 5 bis 10 Kohlenstoffatomen, einen cycloaliphatischen Kohlenwasserstoffrest mit 6 bis 15 Kohlenstoffatomen, einem Xylylenrest oder einen aromatischen, gegebenenfalls Methylsubstituenten und/oder Methylenbrücken aufweisenden Kohlenwasserstoffrest mit insgesamt 6 bis 15 Kohlenstoffatomen steht,

$R^2$ für einen aliphatischen Kohlenwasserstoffrest mit 1 bis 6, insbesondere 1 bis 4 Kohlenstoffatomen, einen Cyclohexylrest oder einen Phenylrest steht, und n für eine ganze Zahl von 2 bis 4 steht.

Im Rahmen dieser Offenbarung ist es jedoch wesentlich, daß die den Carbamidsäureestern zugrundeliegenden Polyisocyanate $R^1 (NCO)_n$ unter den Spaltbedingungen, d.h. unter den Druckbedingungen im Sumpf der Kolonne (4) einen Siedepunkt aufweisen, der mindestens 10°C, vorzugsweise mindestens 40°C unter oder über dem Siedepunkt des jeweiligen Alkohols $R^2OH$ liegt. Im allgemeinen weisen die Polyisocyanate einen höheren Siedepunkt als die Alkohole auf, so daß die Polyisocyanate als Fraktion I am unteren Ende des Dephlegmators (5) anfallen. Im umgekehrten Fall würde natürlich die Alkoholkomponente II als untere Fraktion und die Polyisocyanatkomponente I als obere Fraktion anfallen.

Besonders bevorzugt beim erfindungsgemäßen Verfahren einzusetzende Carbamidsäureester sind solche der allgemeinen Formel

$R^1(NHCOOR^2)_2$

für welche

$R^1$ für den die Isocyanatgruppen von 1,6-Diisocyanatohexan, 1-Isocyanato-3,3,5-trimethyl-5-isocyanatomethyl-cyclohexan, 2,4-Diisocyanato-toluol, 2,6-Diisocyanatotoluol, 2,2'-, 2,4'-oder 4,4'-Diisocyanatodiphenylmethan, 2,4'-oder 4,4'-Diisocyanatodicyclohexylmethan oder 1,5-Diisocyanatonaphthalin verknüpfenden Kohlenwasserstoffrest steht und

$R^2$ für einen Alkylrest mit 1 bis 4 Kohlenstoffatomen steht.

Beispiele für geeignete Carbamidsäureester sind

1-(Butoxycarbonylamino)-3,3,5-trimethyl-5-(butoxycarbonylamino-methyl)-cyclohexan

1-(Methoxycarbonylamino)-3,3,5-trimethyl-5-(methoxycarbonylamino-methyl)-cyclohexan

1-Methyl-2,4-bis-(methoxycarbonylamino)-benzol

1-Methyl-2,6-bis-(methoxycarbonylamino)-benzol

1-Methyl-2,4-bis-(butoxycarbonylamino)-benzol

1-Methyl-2,6-bis-(butoxycarbonylamino)-benzol
1,10-Bis-(methoxycarbonylamino)-decan
1,12-Bis-(butoxycarbonylamino)-dodecan
1,12-Bis-(methoxycarbonylamino)-dodecan
1,12-Bis-(phenoxycarbonylamino)-dodecan
1,3-Bis-(ethoxycarbonylamino-methyl)-benzol
1,3-Bis-(methoxycarbonylamino)-benzol
1,3-Bis-[(methoxycarbonylamino)-methyl]-benzol
1,3,6-Tris-(methoxycarbonylamino)-hexan
1,3,6-Tris-(phenoxycarbonylamino)-hexan
1,4-Bis-(ethoxycarbonylamino)-butan
1,4-Bis-(ethoxycarbonylamino)-cyclohexan
1,5-Bis-(butoxycarbonylamino)-naphthalin
1,6-Bis-(methoxycarbonylamino)-hexan
1,6-Bis-(ethoxycarbonylamino)-hexan
1,6-Bis-(butoxycarbonylamino)-hexan
1,5-Bis-(methoxycarbonylamino)-pentan
1,6-Bis-(methoxymethylcarbonylamino)-hexan
1,8-Bis-(ethoxycarbonylamino)-octan
1,8-Bis-(phenoxycarbonylamino)-4-(phenoxycarbonylaminmethyl)-octan
2,2'-Bis-(4-propoxycarbonylamino-phenyl)-propan
2,4'-Bis-(ethoxycarbanylamino)-diphenylmethan
2,4-Bis-(methoxycarbonylamino)-cyclohexan
4,4'-Bis-(ethoxycarbonylamino)-dicyclohexylmethan
2,4'-Bis-(ethoxycarbonylamino)-diphenylmethan
4,4'-Bis-(methoxycarbonylamino)-2,2'-dicyclohexylpropan
4,4'-Bis-(methoxycarbonylamino)-biphenyl
4,4'-Bis-(butoxycarbonylamino)-2,2'-dicyclohexylpropan
4.4'-Bis-(phenoxycarbonylamino)-dicyclohexylmethan
4,4'-Bis-(phenoxycarbonylamino)-diphenylmethan

Bei den genannten "Butoxy-Resten" handelt es sich immer um n-Butoxy-Reste.

Als Hochsieder für die Durchführung des erfindungsgemäßen Verfahrens kommen Flüssigkeiten oder Feststoffe in Betracht, deren Siedepunkte unter den Druckbedingungen im Sumpf der Kolonne (4) mindestens 10°C, vorzugsweise mindestens 40°C oberhalb der Siedepunkte der dem zu spaltenden Carbamidsäureestern zugrundeliegenden Isocyanate und Alkohole liegen und die im übrigen folgende Bedingungen erfüllen:

a) Sie müssen unter den Spaltbedingungen weitgehend sowohl die als Ausgangsmaterialien eingesetzten Carbamidsäureester als auch die als Reaktionsnebenprodukte anfallenden Isocyanatfolgeprodukte lösen,

b) sie müssen unter den Spaltbedingungen thermisch weitgehend stabil sein,

c) sie müssen gegenüber den eingesetzten Carbamidsäureestern und den entstehenden Isocyanaten weitgehend chemisch inert sein,

d) sie müssen unter den Spaltbedingungen weitgehend destillierbar sein,

e) sie müssen sich destillativ von den Reaktionsnebenprodukten weitgehend abtrennen lassen,

f) sie müssen weitgehend recyclisierbar sein.

Diesen Forderungen entsprechend kommen als erfindungsgemäß verwendbare Hochsieder beispielsweise die in US-PS 3 919 278, Kolonne 3, Zeilen 28 bis 50 erwähnten Kohlenwasserstoffe, außerdem beispielsweise die verschiedenen, isomeren Benzyltoluole, Terphenyle, Phthalsäure-di(ar)alkylester, o-Phosphorsäure-tri(ar)alkylester mit jeweils 1 bis 10 Kohlenstoffatomen in den (Ar)alkylresten oder beliebige Gemische derartiger Verbindungen in Betracht. Besonders bevorzugt werden technisches Dibenzyltoluol, Benzyl-n-butyl-phthalat oder technisches Terphenyl eingesetzt.

Bei der Durchführung des erfindungsgemäßen Verfahrens kommen 5- bis 90-gew.-%ige, vorzugsweise 50- bis 80-gew.-%ige Lösungen der Carbamidsäureester in einem der beispielhaft genannten Hochsieder oder einem Gemisch derselben zum Einsatz.

Diese Lösungen werden im Wärmetauscher (3) unter einem Druck, der oberhalb des Spaltdrucks und vorzugsweise bei 3 bis 100 bar liegt, auf eine Temperatur von 100 bis 300°C erhitzt und kontinuierlich als Seitenstrom in die Sumpf-beheizte Spaltkolonne geleitet bzw. entspannt. Die Eintragstelle befindet sich vorzugsweise in der unteren Hälfte der Kolonne, jedoch oberhalb des Sumpfes.

Die für das erfindungsgemäße Verfahren geeigneten Spaltkolonnen entsprechen herkömmlichen Destillationskolonnen in diversen Ausführungsformen und können sehr unterschiedlich konstruiert sein, Die Kolonne kann mit Füllkörpern der verschiedensten Arten gefüllt sein, beispielsweise Raschig-Ringen oder Gewebepackungen aus Metall oder Glas oder kann Trennböden, wie Glockenböden, enthalten, Sie muß lediglich so betrieben werden können, daß die in die Spaltkolonne eingespeisten Carbamidsäureester sich gleichmäßig verteilen und die gasförmigen/flüssigen Produkte/Hochsieder aus der Destillation entnommen werden können, Die Einspeisung des Seitenstroms erfolgt über eine geeignete Vorrichtung, beispielsweise über eine Düse oder ein Druckhalteventil.

Während der Durchführung des erfindungsgemäßen Verfahrens liegt der im Sumpf der Kolonne gemessene Druck bei 0,001 bis 5 bar, vorzugsweise bei 10 bis 500 mbar. Die Temperatur im Sumpf der Kolonne liegt bei 200 bis 300°C. Temperatur- und Druckbedingungen werden im übrigen innerhalb dieser Bereiche so eingestellt, daß der verwendete Hochsieder im Sumpf siedet und in der Kolonne von der Eintragsstelle des Seitenstroms nach oben hin eine kontinuierliche Verarmung an Carbamidsäureester erfolgt.

Die Spaltprodukte werden am Kopf der Kolonne selektiv kondensiert, wobei im allgemeinen das Polyisocyanat mit dem höheren Siedepunkt im Dephlegmator (5) kondensiert und als Seitenstrom I in (9) gesammelt wird, während die Alkoholkomponente mit dem niedrigeren Siedepunkt erst im Kondensator (6) kondensiert und als Fraktion II im Behälter (10) gesammelt wird. Erforderlichenfalls können selbstverständlich die Ströme I und/oder II einer destillativen Aufarbeitung unterzogen werden, wobei insbesondere die Fraktion I von mitgerissenem Hochsieder befreit wird, der als Rückstand der Destillation gesammelt und zur Herstellung der Ausgangslösung wiederverwendet werden kann. Im allgemeinen bestehen die Fraktionen I bzw. II zu mindestens zu 90 % aus Polyisocyanat $R^1(NCO)_n$ bzw. Alkohol $R^2OH$.

Gleichzeitig wird kontinuierlich beispielsweise über einen Sumpfüberlauf eine Menge an Hochsieder ausgeschleust, die in erster Näherung der Menge des mit der Carbamidsäureester-Lösung eingebrachten Hochsieders entspricht. Der über den Sumpfablauf, d.h. über den Sumpfüberlauf ausgeschleuste, beispielsweise im Sumpfablaufbehälter (8) gesammelte Hochsieder enthält außerdem geringe Mengen an schwerflüchtigen Reaktionsnebenprodukten, so daß oftmals vor der Wiederverwendung des ausgeschleusten Hochsieders dessen destillative Aufarbeitung angezeigt ist.

Es ist ein besonderer Vorteil des erfindungsgemäßen Verfahrens, daß die bei der Spaltungsreaktion entstehenden Polyisocyanate schnell in gasförmiger Form aus der Reaktionszone entfernt, dabei destillativ gereinigt und nur einer geringen thermischen Belastung ausgesetzt werden. Dies hat zur Folge, daß die an sich bekannten Folgereaktionen von Isocyanaten weitgehend unterdrückt werden, so daß ein hoher Reinheitsgrad und eine hohe Ausbeute an Verfahrensprodukt (Polyisocyanat) von über 90 % resultieren.

In den nachfolgenden Beispielen beziehen sich alle Prozentangaben auf das Gewicht.

Beispiele 1 bis 9

Beschreibung der Apparatur (vgl. Zeichnung)

Als Spaltkolone (4) dient eine evakuierte Doppelmantelkolonne aus Glas mit einem Durchmesser von 25 mm und einer effektiven Länge von 400 mm, die mit Raschigringen aus Glas (6 x 6 mm) gefüllt ist. Die Einspeisung der Lösung des Carbamidsäureesters erfolgt in der unteren Hälfte der Kolonne über eine beheizte Pumpe (2) und einen Wärmeaustauscher (3) durch ein Druckhalteventil (nicht gezeichnet), das auf 5 bar eingestellt ist. Die Ausschleusung des Hochsieders und der Reaktionsnebenprodukte erfolgt durch Überlauf des Kolonnensumpfes. Die Entnahme der Spaltprodukte erfolgt am Kopf der Kolonne über zwei Entnahmeböden unterhalb des Dephlegmators (5) und zwischen Dephlegmator und Kondensator (6). In allen Beispielen weisen die Alkohole einen tieferen Siedepunkt als die entstehenden Diisocyanate auf.

Durchführung:

Die Reaktionsbedingungen, analytischen Daten und Ausbeuten an Verfahrensprodukten sind in nachstehender Tabelle zusammengestellt. Zur Durchführung des Verfahrens wird jeweils der Sumpf der Kolonne mit 100 g Hochsieder gefüllt. Der Hochsieder wird dann jeweils unter einem entsprechenden Vakuum auf die Spalttemperatur erhitzt. In die Spaltkolonne werden innerhalb 6 Stunden 600 g einer Lösung von Bisurethan im Hochsieder eindosiert. Nach 4 Stunden Laufzeit befindet sich die Kolonne im Gleichgewichtszustand. Dann wird am unteren Entnahmeboden Fraktion I abgenommen, am darüber angeordneten Entnahmeboden Fraktion II. Die Ausbeute bezieht sich jeweils auf das in Fraktion I vorliegende Diisocyanat. Die analytischen Untersuchungen erfolgten mittels SFC (Supercritical Fluid Chromatography).

Abkürzungen:

| | |
|---|---|
| MIPDU: | 1-(Methoxycarbonylamino)-3,3,5-trimethyl-5-(methoxycarbonylamino-methyl)-cyclohexan |
| MHDU: | 1,6-Bis-(methoxycarbonylamino)-hexan |
| BHDU: | 1,6-Bis-(n-butoxycarbonylamino)-hexan |
| MTDU: | 1-Methyl-2,4-bis-(methoxycarbonylamino)-benzol |
| DBT: | Dibenzyltoluol (technisches Isomerengemisch) |
| BBP: | Benzyl-n-butyl-phthalat |
| TER: | techn. Terphenyl |
| Monoisocyanat: | teilgespaltenes, Urethan- und Isocyanatgruppen aufweisendes Zwischenprodukt |

| Beispiel-Nr. | | 1 | 2 | 3 | 4 | 5 | 6 | 7 | 8 | 9 |
|---|---|---|---|---|---|---|---|---|---|---|
| Bisurethan | | MIPDU | MIPDU | MHDU | MHDU | BHDU | BHDU | BHDU | MTDU | MTDU |
| Hochsieder | | DBT | BBP | DBT | BBP | DBT | BBP | TER | DBT | TER |
| Konzentration | % | 60 | 60 | 60 | 60 | 60 | 60 | 60 | 60 | 60 |
| Temperatur, Sumpf | °C | 260 | 260 | 260 | 260 | 260 | 260 | 260 | 240 | 230 |
| Druck, Sumpf | mbar | 35 | 50 | 35 | 50 | 35 | 50 | 35 | 20 | 25 |
| Kondensator | °C | -78 | -78 | -78 | -78 | 30 | 30 | 30 | -78 | -78 |
| Dephlegmator | °C | 80 | 80 | 80 | 80 | 110 | 110 | 110 | 80 | 80 |
| Auswaage Fraktion I | g/h | 12,1 | 11,4 | 14,4 | 14,6 | 16,7 | 15,1 | 14,6 | 12,1 | 10,6 |
| Fraktion II | g/h | 47,1 | 47,4 | 42,6 | 43,0 | 42,7 | 43,6 | 44,7 | 36,5 | 37,4 |
| Sumpfüberlauf | g/h | 40,2 | 41,2 | 40,7 | 41,6 | 40,5 | 41,3 | 40,7 | 50,6 | 51,0 |
| Fraktion II Alkohol | % | 99,6 | 99,2 | 99,9 | 99,1 | 94,5 | 94,6 | 95,3 | 99,8 | 99,3 |
| Bisurethan | % | 0,4 | 0,6 | 0,1 | 0,9 | 5,5 | 5,4 | 4,7 | 0,2 | 0,7 |
| Analytik Fraktion I Diisocyanat | % | 93,6 | 94,6 | 97,6 | 95,9 | 94,4 | 91,8 | 92,1 | 94,0 | 92,2 |
| Monoisocyanat | % | 1,8 | 2,1 | 2,0 | 2,7 | 4,0 | 1,5 | 2,3 | 3,6 | 4,3 |
| Bisurethan | % | 0,0 | 0,7 | 0,0 | 0,2 | 1,3 | 5,3 | 1,8 | 1,7 | 0,2 |
| Hochsieder | % | 1,9 | 2,6 | 0,4 | 1,2 | 0,3 | 1,4 | 3,8 | 0,7 | 3,3 |
| Sumpf-überlauf Diisocyanat | % | 0,0 | 0,1 | 0,0 | 0,1 | 0,2 | 0,2 | 0,1 | 0,2 | 0,3 |
| Monoisocyanat | % | 0,0 | 0,1 | 0,0 | 0,1 | 0,1 | 0,0 | 0,2 | 0,0 | 0,1 |
| Bisurethan | % | 0,1 | 0,0 | 0,1 | 0,5 | 0,3 | 0,2 | 0,2 | 0,3 | 0,1 |
| Hochsieder | % | 99,4 | 97,2 | 98,3 | 96,1 | 98,8 | 96,9 | 98,2 | 98,9 | 98,0 |
| Nebenprodukte | % | 0,5 | 2,6 | 1,6 | 3,2 | 0,6 | 2,7 | 1,3 | 0,6 | 1,5 |
| Ausbeute | %d.Th. | 97,4 | 96,3 | 95,6 | 94,9 | 92,8 | 92,1 | 94,7 | 93,8 | 94,3 |

**Patentansprüche**

1. Verfahren zur kontinuierlichen, katalysatorfreien Herstellung von Polyisocyanaten durch thermische Spaltung der den Polyisocyanaten entsprechenden N-substituierten Carbamidsäureester bei 200° C bis 300°C in einer Sumpf-beheizten, als Spaltreaktor dienenden Destillationskolonne unter anschließender Auftrennung der Spaltprodukte in eine, vorwiegend aus der Isocyanatkomponente bestehende Fraktion I und eine vorwiegend aus der Alkoholkomponente der Carbamidsäureester bestehende Fraktion II, wobei die Spaltung in Gegenwart eines Hochsieders durchgeführt wird, der für die Carbamidsäureester ein Lösungsmittel darstellt, gegenüber den Carbamidsäureestern und den Spaltprodukten inert ist, unter den Druck- und Temperaturbedingungen im Sumpf der Destillationskolonne siedet und unter diesen Druckbedingungen einen Siedepunkt aufweist, der mindestens 10°C über dem Siedepunkt beider Spaltprodukte liegt, dadurch gekennzeichnet, daß die zu spaltenden Carbamidsäureester in Form einer 5 bis 90-gew.-%igen Lösung oberhalb des Spaltdrucks auf eine Temperatur innerhalb des Bereichs von 100°C bis 300°C, jedoch eine Temperatur, die oberhalb des Schmelzpunktes des Polyurethans liegt, erhitzt werden und anschließend unter Entspannung als Seitenstrom in die Destillationskolonne (4) geleitet werden, in deren Sumpf ein Druck innerhalb des Bereichs von 0,001 bis 5 bar und eine Temperatur innerhalb des Bereichs von 200°C bis 300°C so aufrecht erhalten werden, daß der Hochsieder im Sumpf am Sieden gehalten wird, wobei gleichzeitig die Spaltprodukte am Kopf der Destillationskolonne in Form der Fraktionen I und II kontinuierlich selektiv kondensiert werden und über den Sumpfablauf kontinuierlich eine solche Menge des gegebenenfalls Verunreinigungen enthaltenden Hochsieders ausgeschleust wird, die in erster Näherung der Menge des Hochsieders entspricht, der als Lösungsmittel für den Carbamidsäureester in die Kolonne eingespeist wird.

2. Verfahren gemäß Anspruch 1, dadurch gekennzeichnet, daß die Fraktionen I und II separat einer Feindestillation unterzogen werden, wobei die Destillationsrückstände dieser Feindestillationen zusammen mit der in die Destillationskolonne einzuspeisenden Carbamidsäurelösung in die Destillationskolonne zurückgeführt werden.

3. Verfahren gemäß Anspruch 1 und 2, dadurch gekennzeichnet, daß der Sumpf-Ablauf destillativ aufgearbeitet wird, und der dabei als Destillat anfallende Hochsieder als Lösungsmittel für die zu spaltenden Carbamidsäureester wieder verwendet wird.

4. Verfahren gemäß Anspruch 1 und 3, dadurch gekennzeichnet, daß man als Carbamidsäureester mindestens eine Verbindung einsetzt, die der Formel

$R^1 (NHCOOR^2)_2$

entspricht, wobei

$R^1$ für den die Isocyanatgruppen von 1,6-Diisocyanatohexan, 1-Isocyanato-3,3,5-trimethyl-5-isocyanatomethyl-cyclohexan, 2,4-Diisocyanatotoluol, 2,6-Diisocyanatotoluol, 2,2'-, 2,4'- oder 4,4'-Diisocyanatodiphenylmethan, 2,4'- oder 4,4'-Diisocyanatodicyclohexylmethan oder 1,5-Diisocyanatonaphthalin verknüpfenden Kohlenwasserstoffrest steht und

$R^2$ für einen Alkylrest mit 1 bis 4 Kohlenstoffatomen steht.

5. Verfahren gemäß Anspruch 1 bis 4, dadurch gekennzeichnet, daß man als Carbamidsäureester die Bis-O-alkylurethane des 1,6-Diisocyanatohexans, 1-Isocyanato-3,3,5-trimethyl-5-isocyanatomethyl-cyclohexan, 2,4-Diisocyanatotoluol oder dessen Gemischen mit 2,6-Diisocyanatotoluol mit jeweils 1 bis 4 Kohlenstoffatomen im Alkylrest verwendet.

6. Verfahren gemäß Anspruch 1 bis 5, dadurch gekennzeichnet, daß man als Hochsieder Benzyltoluole, Terphenyle, Phthalsäuredi(ar)alkylester oder o-Phosphorsäuredi(ar)trialkylester jeweils mit 1 bis 10 Kohlenstoffatome enthaltenden (Ar)alkylresten verwendet.

**Claims**

1. A process for the continuous catalyst-free production of polyisocyanates by thermal decomposition of the N-substituted carbamic acid esters corresponding to the polyisocyanates at 200 to 300°C in a sump-heated distillation column serving as decomposition reactor with subsequent separation of the decomposition products into a fraction I consisting predominantly of the isocyanate component and a fraction II consisting predominantly of the alcohol component of the carbamic acid ester, the decomposition reaction being carried out in the presence of a high boiler which is a solvent for the carbamic acid esters, is inert to the carbamic acid esters and the decomposition products, boils under the pressure and temperature conditions prevailing in the sump of the distillation column and has a boiling point under those pressure conditions which is at least 10°C above the boiling point of both decomposition products, characterized in that the carbamic acid esters to be decomposed - in the form of a 5 to 90% by weight solution - are heated above the decomposition pressure to a temperature of 100 to 300°C, but to a temperature above the melting point of the polyurethane, and are subsequently introduced with expansion as a sidestream into the distillation column (4), in the sump of which a pressure of 0.001 to 5 bar and a temperature of 200 to 300°C are maintained so that the high boiler is kept boiling in the sump, the decomposition products simultaneously being condensed continuously and selectively at the head of the distillation column in the form of fractions I and II and the high boiler optionally containing impurities is continuously removed *via* the sump outlet in a quantity substantially corresponding to the quantity of high boiler introduced into the column as solvent for the carbamic acid ester.

2. A process as claimed in claim 1, characterized in that fractions I and II are separately subjected to fine distillation, the distillation residues of this fine distillation being returned to the distillation column together with the carbamic acid ester solution to be introduced into the distillation column.

3. A process as claimed in claims 1 and 2, characterized in that the sump outflow is worked up by distillation and the high boiler accumulating as distillate is reused as solvent for the carbamic acid esters to be split.

4. A process as claimed in claims 1 and 3, characterized in that at least one compound corresponding to the formula

$R^1(NHCOOR^2)_2$

in which
R¹    is the hydrocarbon radical linking the isocyanate groups of 1,6-diisocyanatohexane, 1-isocyanato-3,3,5-trimethyl-5-isocyanatomethyl cyclohexane, 2,4-diisocyanatotoluene, 2,6-diisocyanatotoluene, 2,2'-, 2,4'- or 4,4'-diisocyanatodiphenyl methane, 2,4'- or 4,4'-diisocyanatodicyclohexyl methane or 1,5-diisocyanatonaphthalene and
R²    is a $C_{1-4}$ alkyl radical,
      is used as the carbamic acid ester.

5. A process as claimed in claims 1 to 4, characterized in that the bis-O-alkyl urethanes of 1,6-diisocyanatohexane, 1-isocyanato-3,3,5-trimethyl-5-isocyanatomethyl cyclohexane, 2,4-diisocyanatotoluene or mixtures thereof with 2,6-diisocyanatotoluene containing 1 to 4 carbon atoms in the alkyl group are used as the carbamic acid esters.

6. A process as claimed in claims 1 to 5, characterized in that benzyl toluenes, terphenyls, phthalic acid di(ar)alkyl esters or o-phosphoric acid di(ar)trialkyl esters containing $C_{1-10}$ (ar)alkyl groups are used as the high boilers.

**Revendications**

1. Procédé pour la préparation continue sans catalyseur de polyisocyanates par dissociation thermique des carbamates N-substitués correspondants aux polyisocyanates à une température de 200°C à 300°C dans une colonne de distillation chauffée en queue, servant de réacteur de dissociations, avec séparation subséquente des produits de la dissociation en une coupe I essentiellement constituée des constituants d'isocyanates et une coupe II essentiellement constituée des constituants d'alcools des carbamates, la dissociation étant réalisée en présence d'un constituant de point d'ébullition élevé qui est un solvant pour les carbamates, est inerte par rapport aux carbamates et aux produits de la dissociation, bout dans les conditions de pression et de température en queue de la colonne de distillation et présente dans ces conditions de pression un point d'ébullition qui se trouve au moins 10°C au-dessus du point d'ébullition des deux produits de la dissociation, caractérisé en ce qu'on chauffe les carbamates à dissocier dans la forme d'une solution de 5 à 90 % en poids au-dessus de la pression de dissociation à une température à l'intérieur du domaine de 100°C à 300°C, cependant à une température qui se trouve au-dessus du point de fusion du polyuréthane et qu'on les introduit ensuite sous décompression en tant que courant latéral dans la colonne de distillation (4), dans la queue de laquelle on maintient une pression à l'intérieur du domaine de 0,001 à 5 bars et une température à l'intérieur du domaine de 200°C à 300°C, de sorte que le constituant de point d'ébullition élevé est maintenu en ébullition en queue, simultanément les produits de la dissociation étant sélectivement condensés en continu en tête de la colonne de distillation dans la forme des coupes I et II et par l'intermédiaire de l'écoulement de queue étant extraite en continu une telle quantité du constituant de point d'ébullition élevé contenant éventuellement des impuretés qui correspond en première approximation à la quantité du constituant de point d'ébullition élevé qui est introduite dans la colonne en tant que solvant pour le carbamate.

2. Procédé selon la revendication 1, caractérisé en ce qu'on soumet les coupes I et II séparément à une distillation de précision, les résidus de distillation de ces distillations de précision étant renvoyés ensemble dans la colonne de distillation avec la solution de carbamate à introduire dans la colonne de distillation.

3. Procédé selon les revendications 1 et 2, caractérisé en ce qu'on traite par distillation l'écoulement de queue et en ce qu'on réutilise ce faisant le constituant de point d'ébullition élevé produit en tant que distillat comme solvant pour les carbamates à dissocier.

4. Procédé selon les revendications 1 et 3, caractérisé en ce qu'on utilise en tant que carbamate au moins un composé qui correspond à la formule

$R^1 (NHCOOR^2)_2$

dans laquelle

$R^1$ représente le résidu d'hydrocarbure lié aux groupes isocyanates du 1,6-diisocyanatohexane, du 1-isocyanato-3,3,5-triméthyl-5-isocyanatométhylcyclohexane, du 2,4-diisocyanatotoluène, du 2,6-diisocyanatotoluène, du 2,2'-, du 2,4'- ou du 4,4'-diisocyanatodiphénylméthane, du 2,4'- ou du 4,4'-diisocyanatodicyclohexylméthane ou du 1,5-diisocyanatonaphtalène et

$R^2$ représente un résidu alkyle avec de 1 à 4 atomes de carbone.

5. Procédé selon l'une quelconque des revendications 1 à 4, caractérisé en ce qu'on utilise en tant que carbamate les bis-o-alkyluréthanes du 1,6-diisocyanatohexane, du 1-isocyanato-3,3,5-triméthyl-5-isocyanatométhylcyclohexane, du 2,4-diisocyanatotoluène ou des mélanges de ceux-ci avec du 2,6-diisocyanatotoluène présentant à chaque fois de 1 à 4 atomes de carbone dans le résidu alkyle.

6. Procédé selon l'une quelconque des revendications 1 à 5, caractérisé en ce qu'on utilise en tant que constituant de point d'ébullition élevé des benzyltoluènes, des triphényles, des phtalates de di(ar)alkyles ou des o-phosphates de tri(ar)alkyles avec à chaque fois des résidus (ar)alkyles contenant de 1 à 10 atomes de carbone.

FIG.1